Europäisches Patentamt

European Patent Office

Office européen des brevets

(11) Publication number: **0 215 956**

**A1**

(12)

# EUROPEAN PATENT APPLICATION
published in accordance with Art. 158(3) EPC

(21) Application number: 86902015.6

(22) Date of filing: 14.03.86

Data of the international application taken as a basis:

(86) International application number:
PCT/JP86/00129

(87) International publication number:
WO86/05395 (25.09.86 86/21)

(51) Int. Cl.⁴: **A 61 K 31/59**

(30) Priority: 14.03.85 JP 51410/85

(43) Date of publication of application:
01.04.87 Bulletin 87/14

(84) Designated Contracting States:
DE FR IT SE

(71) Applicant: Chugai Seiyaku Kabushiki Kaisha
5-1, 5-chome, Ukima Kita-ku
Tokyo(JP)

(72) Inventor: MORIMOTO, Shigeto 5-14, Seiwadai 2-chome
Kawaicho, Kitakatsuragi-gun
Nara, 636(JP)

(74) Representative: Vossius & Partner
Siebertstrasse 4 P.O. Box 86 07 67
D-8000 München 86(DE)

(54) COMPOSITION FOR TREATING SKIN DISEASE.

(57) A composition for oral administration containing as an effective ingredient vitamin $D_3$ hydroxylated at 1α-position which is used for treating inflammatory keratosis such as psoriasis and abnormal keratosis. Preferable vitamin $D_3$'s hydroxylated at 1α-hydroxyvitamin $D_3$ and 1α,25-dihydroxyvitamin $D_3$. Psoriasis can be markedly cured by oral administration of this composition.

EP 0 215 956 A1

SPECIFICATION

PHARMACEUTICAL COMPOSITION FOR

TREATING DERMAL DISEASES

Technical Field

The present invention relates to a pharmaceutical composition for oral administration that has as its effective component at least one vitamin $D_3$ hydroxylated at $1\alpha$-position and which is intended to treat inflammatory keratosis (e.g. psoriasis) and keratonosis.

Background of the Invention

Inflammatory keratosis typified by psoriasis is one of the commonly experienced chronic or refractory inflammatory dermal diseases but the cause of this lesion has not been clearly identified. Treatments currently applied to treat inflammatory keratosis include: Goeckerman treatment wherein the involved areas are covered with coal tar ointment and subsequently irradiated with ultraviolet [North West Med., 24, 2 - 9 (1925)], PUVA treatment wherein administration of psoralen is followed by exposure to ultraviolet radiation of long wavelength [N. Eng. J. Med., 291, 1207 - 1211 (1974)], external application of corticosteroid [Science, 197, 994 - 996 (1977)], administration of antitumor methotrexate [Arch. Dermatol., 95, 2 - 11 (1967)], Ingram treatment wherein the affected sites are covered with anthralin ointment, then painted with a solution of coal tar and finally irradiated with ultraviolet [Br. Med. J., 2, 591 - 594 (1953)], both oral administration and external use of vitamin A derivatives [JAMA., 207, 1863 - 1868 (1969)] and Dermatologica, 157, 38 - 44 (1978)], and dialysis [JAMA., 240, 1270 (1978)]. However, psoriasis is often refractory to these treatments. In addition, some of these treatments cause side effects whose degree is by no means negligible. For instance, cases have been known where skin cancer occurred after exposure to ultraviolet radiation.

It is well known that in vivo hydroxylated vitamin $D_3$ metabolites such as 25-hydroxyvitamin $D_3$, 24R,25-dihydroxyvitamin $D_3$ and $1\alpha$,25-dihydroxyvitamin $D_3$, and $1\alpha$,25-dihydroxyvitamin $D_3$ and its synthetic analog

1α-hydroxyvitamin $D_3$, in particular, have a strong ability to absorb intestinal calcium and to mobilize bone salt and hence are useful as therapeutic agents for various diseases caused by abnormal calcium metabolism, and that they also have a strong capacity to induce the differentiation of human or mouse bone marrow leukemia cells [Proc. Natl. Acad. Sci., USA, 78, 4990 - 4994 (1980) and Biochem. Biophys. Res. Commun., 102, 937 - 943 (1981)]. It has also been shown that 1α-hydroxyvitamin $D_3$ and 1α,25-dihydroxyvitamin $D_3$ can be used as therapeutic agents for psoriasis and other dermal diseases by topical application (European Patent Publication No. 129003). The present inventors reviewed the topical use of 1α-hydroxyvitamin $D_3$ and other vitamin $D_3$ compounds in the form of drugs for external use such as ointments as disclosed in EPP 129003. As a result, the inventors have found that the 1α-hydroxyvitamin $D_3$ compounds are not metabolized and have a tendency to accumulate under the skin in their active form. Dermal diseases such as psoriasis are seldom cured by application of a single dose of 1α-hydroxyvitamin $D_3$ or other drugs and even if the disease is slight in degree it is necessary for the drug to be administered continuously for at least two weeks. If the disease is serious, the drug application has to be continued for several weeks to several months. However, as already mentioned, vitamin $D_3$ compounds administered as drugs for external use have a tendency to accumulate under the skin without being metabolized. In addition, the vitamin $D_3$ compounds having a hydroxyl group at 1α position which are intended to be used in the present invention are so toxic that it is not recommended that they are used topically for a prolonged period without interruption. This is well supported by toxicological data shown later in this specification that demonstrate that the toxicity of 1α-hydroxyvitamin $D_3$ compounds as directly administered under the skin was at least about 10 times greater than when they were administered perorally. Therefore, topical administration of 1α-hydroxyvitamin $D_3$ compounds as a therapeutic agent for psoriasis is effective if their use is limited to a short period of time but the compounds are not necessarily suitable for continuous use over a prolonged period if the disease is serious. Therapeutically, topical application of 1α-hydroxyvitamin $D_3$ compounds has the additional disadvantage that they have to be applied to a wide area if the site affected by the lesion is extensive.

Under the circumstances described above, the present inventors made concerted efforts to develop an effective method for treating psoriasis and found that known vitamin $D_3$ compounds having a hydroxyl group at 1α position as typified by 1α-hydroxyvitamin $D_3$ and 1α,25-dihydroxyvitamin $D_3$ would provide excellent therapeutic effects even when they were administered perorally. Orally administered 1α-hydroxyvitamin $D_3$ compounds would be adsorbed by the small intestines and, unlike the same compounds administered topically, would be metabolized in the living body without being accumulated in their active form. Consequently, as already mentioned, perorally administered 1α-hydroxyvitamin $D_3$ compounds will present lower toxic effects than when they are administered topically. Another advantage of administering 1α-hydroxyvitamin $D_3$ compounds perorally is that because of the rapidity with which they influence the serum calcium level, their dose level can be precisely controlled by monitoring the serum calcium level at intervals after administration of the compounds. Needless to say, an extensively affected area of lesion can be effectively treated by peroral administration of 1α-hydroxyvitamin $D_3$ compounds and the cumbersomeness of topical application of the compounds is entirely eliminated.

Disclosure of the Invention

The present invention relates to a pharmaceutical composition that has an effective amount of at least one vitamin $D_3$ compound having a hydroxyl group at 1α position and which is intended to be administered perorally for treating inflammatory keratosis and keratonosis.

Inflammatory keratosis and keratonosis which are intended to be treated by the pharmaceutical composition of the present invention are chronic and refractory dermal diseases as typified by psoriasis and include the following specific diseases: psoriasis vulgaris, parapsoriasis, lichen ruber, pityriasis rubra pilaris, pityriasis rosea Gibert, pustulosis palmaris et plantaris, ichthyosis vulgaris and morbus Darier.

Specific examples of the vitamin $D_3$ compounds which have a hydroxyl group at 1α position include: metabolites of vitamin $D_3$ such as 1α,25-dihydroxyvitamin $D_3$ and 1α,24,25-trihydroxyvitamin $D_3$; and synthetic analogs thereof such as 1α-hydroxyvitamin $D_3$, 1α,24-dihydroxyvitamin $D_3$, 24,24-difluoro-1α,25-dihydroxyvitamin $D_3$, and 26,26,26,27,27,27-hexafluoro-1α,25-dihydroxyvitamin $D_3$. From the viewpoint of providing high therapeutic effects and ease of commercial production, 1α,25-dihydroxyvitamin $D_3$, 1α-hydroxyvitamin $D_3$ and 1α,24-dihydroxyvitamin $D_3$ are particularly useful drugs for attaining the purpose of the present invention.

The vitamin $D_3$ compounds listed above are known and may be prepared by the methods described in Japanese Patent Public Disclosure Nos. 62750/1973, 95956/1974, 108047/1976, 42864/1977 and 111460/1980, and Japanese Patent Publication No. 500864/1984.

These vitamin $D_3$ compounds are formulated in dosage forms suitable for peroral administration such as tablets and capsules. Since these vitamin $D_3$ compounds exhibit strong effects on the serum calcium level when they are administered perorally, the single dosage of these compounds is limited to a trace level and this requires a particular need for achieving uniformity in the contents of the compounds in dosage forms. Therefore, it is preferable to use these vitamin $D_3$ compounds as oily solutions after they have been dissolved in oil bases. Dosage forms which are particularly suitable for peroral administration can be attained by incorporating the oily solutions in soft capsules. Suitable oil bases are those which are oily at room temperature and include triglycerides of aliphatic acids, fats and oils, middle-chain unsaturated aliphatic acids such as linoleic acid, coconut oil, olive oil, corn oil and soybean oil.

The vitamin $D_3$ compounds having a hydroxyl group at

1α position are preferably administered in such amounts that they will provide blood levels ranging from 20 to 400 pg/ml and dose levels that meet this requirement may be appropriately selected. For a human adult, the dose level typically ranges from 0.5 to 3.0 µg per day. The vitamin $D_3$ compounds having a hydroxyl group at 1α position which are employed as effective compounds in the present invention may be used either individually or in combination with themselves.

Best Mode for Carrying out the Invention

Example 1    Peroral Administration of 1α-Hydroxyvitamin $D_3$

(1)   81-year-old male patient

This patient consulted a doctor in 1982 and his main complaint was pains in the loins due to senile osteoporosis. In order to treat this disease, 0.75 µg of 1α-hydroxyvitamin $D_3$ was orally administered per day. In two months and a half after the start of administration of the drug, the psoriasis vulgaris which the patient had been suffering from over the past 30 years dramatically diminished in severity and a half month later the disease virtually disappeared.

(2)   54-year-old male patient

This patient had been suffering from psoriasis vulgaris for one year, during which period he was under therapy involving external use of corticosteroid. In two months after the start of oral administration of 1.0 µg of 1α-hydroxyvitamin $D_3$ per day, the patient showed a sign of improvement in the dermal lesion condition as manifested by diminished flares and rashes on the skin. Four months later, the dermal lesion condition had improved pronouncedly except that the patient was still suffering from a slight loss of dermal pigment. Thereafter, the external use of corticosteroid was suspended and only 1α-hydroxyvitamin $D_3$ was kept administered perorally. Six months later, the dermal lesion in the patient had completely cured.

(3)   37-year-old male patient

This patient had been suffering from psoriasis vulgaris for 10 years and throughout this period the dermal lesion was localized on his elbows and knees but 3 months before diagnosis the lesion had expanded to cover the whole body except the head and face. Two months after the start of oral administration of 1α-hydroxyvitamin $D_3$ in a daily dose of 1 μg, the lesion had diminished by a small degree and two more months later the patient showed a pronounced improvement in the condition. This improvement could be maintained by subsequent peroral administration of 1α-hydroxyvitamin $D_3$ in a daily dose of 0.5 μg.

(4)  57-year-old male patient

This patient had been suffering from psoriasis vulgaris for 12 years, during which period he was under therapy involving external use of corticosteroid, exposure to ultraviolet radiation and peroral administration of corticosteroid but without any satisfactory results. After suspending the external use of corticosteroid, peroral administration of 1α-hydroxyvitamin $D_3$ in a daily dose of 1 μg was initiated. Two months later, the dermal lesion on the soles had disappeared and the condition of the lesion elsewhere on his body had improved markedly.

(5)  56-year-old male patient

This patient had been suffering from psoriasis vulgaris for 6 years, during which period he was under therapy involving external use of corticosteroid but without any satisfactory results. During the three months after the start of peroral administration of 1α-hydroxyvitamin $D_3$ in a daily dose of 1 μg, the patient saw moderate improvement in the dermal lesion condition.

(6)  56-year-old male patient

This patient had been suffering from psoriasis vulgaris for 25 years, during which period he was under therapy involving external use of corticosteroid but without any satisfactory results. During the three months after the start of peroral administration of 1α-hydroxyvitamin $D_3$ in a daily dose of 1 μg, the patient saw moderate improvement in the dermal lesion condition.

Example 2  Peroral Administration of 1α,25-Dihydroxyvitamin $D_3$

(1)  14-year-old male patient

This patient had been suffering from psoriasis vulgaris for 2 years. The dermal lesion that initially occurred on his head had expanded to cover the whole body 4 months before the patient consulted a doctor. During the three months after the start of peroral administration of $1\alpha,25$-dihydroxyvitamin $D_3$ in a daily dose of 0.5 µg, the patient saw moderate improvement in the dermal lesion condition as manifested by diminished rashes and flares.

Example 3    Side Effects

the six patients under therapy by oral administration of $1\alpha$-dihydroxyvitamin $D_3$ and the one patient receiving orally administered $1\alpha,25$-dihydroxyvitamin $D_3$ were subjected to the following analyses, tests and measurements at intervals of twice a month in the first two months of administration and once a month in the subsequent period: blood analysis for determining the number of erythrocytes, the number of platelets and the percentage of leucocytes; urine analysis for determining the levels of urinary protein, glucose, pH, acetone and urobilinogen and the presence of occult blood; testing liver functions by determining the levels of blood GOT, GPT and total bilirubin; testing kidney functions by determining the levels of blood creatinine and BUN; and the measurement of blood calcium and inorganic phosphate levels. None of the parameters analyzed showed any abnormal change from the values measured prior to the treatment with $1\alpha$-hydroxyvitamin $D_3$ or $1\alpha,25$-dihydroxyvitamin $D_3$. Not a single patient under therapy had any abnormal subjective symptom or objective sign.

Example 4    Toxicological Test

Acute toxicity of $1\alpha$-hydroxyvitamin $D_3$ was evaluated by its peroral and subcutaneous administration to mice and rats. The results are shown in the following table.

Table : $LD_{50}$ of 1α-hydroxyvitamin $D_3$

| Route of adminis-tration | Sex | $LD_{50}$ in mice (μg/kg) | $LD_{50}$ in rats (μg/kg) |
|---|---|---|---|
| Peroral | male | 680 | 620 |
| | female | 710 | 743 |
| subcuta-neous | male | 55 | 56 |
| | female | 58 | 40 |

Notes: $LD_{50}$ was calculated by the Litchfield-Wilcoxon method. The mice tested were 5-week-old ddY mice, and the rats tested were 5-week-old SD-TCL rats.

Example 5    Formulations in Dosage Forms

a)    One milligram of 1α-hydroxyvitamin $D_3$ was dissolved in 60 g of O.D.O. (triglyceride of middle-chain alphatic acid; produced by The Nissin Oil Mills, Ltd.)  After addition of 30 mg of sorbic acid as a stabilizer, the solution was fed into a gelatin-coated soft capsule making machine and soft capsules each containing 1.0 μg of 1α-hydroxyvitamin $D_3$ were prepared by routine procedures.

b)    Soft capsules were prepared as in a) except that 1.0 mg of 1α-hydroxyvitamin $D_3$ was replaced by 1.0 mg of 1α,25-dihydroxyvitamin $D_3$ and that each of the capsules contained 1.0 μg of 1α,25-dihydroxyvitamin $D_3$.

CLAIMS

1.    A pharmaceutical composition for treating inflammatory keratosis and keratonosis by peroral administration thereof, said composition comprising an effective amount of at least one vitamin $D_3$ compound having a hydroxyl group at $1\alpha$ position and a pharmaceutically acceptable carrier.

2.    A pharmaceutical composition according to Claim 1 wherein the diseases included within the scope of said inflammatory keratosis and keratonosis are psoriasis vulgaris, parapsoriasis, lichen ruber, pityriasis rubra pilaris, pityriasis rosea Gibert, pustulosis palmaris et plantaris, ichthyosis vulgaris, and morbus Darier.

3.    A pharmaceutical composition according to Claim 1 wherein said inflammatory keratosis is psoriasis.

4.    A pharmaceutical composition according to Claim 1 wherein said vitamin $D_3$ compound having a hydroxyl group at $1\alpha$ position is $1\alpha$-hydroxyvitamin $D_3$ or $1\alpha,25$-dihydroxyvitamin $D_3$.

5.    A pharmaceutical composition according to Claim 1 wherein the daily dose of said composition is in the range of 0.5 - 3.0 µg per human adult in terms of the vitamin $D_3$ compound having a hydroxyl group at $1\alpha$ position.

# INTERNATIONAL SEARCH REPORT

International Application No. PCT/JP86/00129 0215956

## I. CLASSIFICATION OF SUBJECT MATTER (if several classification symbols apply, indicate all) ³

According to International Patent Classification (IPC) or to both National Classification and IPC

Int.Cl⁴      A61K31/59

## II. FIELDS SEARCHED

| Minimum Documentation Searched ⁴ | |
|---|---|
| Classification System | Classification Symbols |
| IPC | A61K31/59 |

| Documentation Searched other than Minimum Documentation to the Extent that such Documents are Included in the Fields Searched ⁵ |
|---|
| |

## III. DOCUMENTS CONSIDERED TO BE RELEVANT ¹⁴

| Category* | Citation of Document, ¹⁶ with indication, where appropriate, of the relevant passages ¹⁷ | Relevant to Claim No. ¹⁸ |
|---|---|---|
| Y | JP, A, 60-45516 (Kureha Chemical Industry Co., Ltd.) 12 March 1985 (12. 03. 85) (Family: none) | 1 - 5 |
| Y | EP, A2, 129,003 (YISSUM RESEARCH AND DEVELOPMENT COMPANY OF THE HEBREW UNIVERSITY OF JERUSALEM) 20 March 1984 (20. 03. 84) (Family: none) | 1 - 5 |

* Special categories of cited documents: ¹⁵

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search ² | Date of Mailing of this International Search Report ² |
|---|---|
| May 23, 1986 (23. 05. 86) | June 9, 1986 (09. 06. 86) |
| International Searching Authority ¹ | Signature of Authorized Officer ²⁰ |
| Japanese Patent Office | |

Form PCT/ISA/210 (second sheet) (October 1981)